# EUROPEAN PATENT SPECIFICATION

(11) **EP 0 735 995 B1**
(45) Date of publication and mention of the grant of the patent: **28.07.1999**
(21) Application number: 95905332.3
(22) Date of filing: 09.12.1994
(51) Int. Cl.: C07C 2/64, C07C 2/68

(54) **NAPHTHALENE ALKYLATION WITH PARTIAL RARE EARTH EXCHANGED CATALYST**
NAPHTHALIN-ALKYLIERUNG MIT DURCH SELTENE ERDEN TEILWEISE AUSGETAUSCHTEN KATALYSATOREN
ALKYLATION DE NAPHTALENES PAR DES CATALYSEURS A ECHANGE PARTIEL DE TERRES RARES

(30) Priority: 23.12.1993 US 172300
(43) Date of publication of application: 09.10.1996
(73) Proprietor: MOBIL OIL CORPORATION, Fairfax, Virginia 22037-0001 (US)
(72) Inventor: ARDITO, Susan, Chmura, Ocean, NJ 07712 (US); ASHJIAN, Henri, East Brunswick, NJ 08816 (US); DEGNAN, Thomas, Francis, Jr., Moorestown, NJ 08057 (US); HELTON, Terry, Eugene, Glen Mills, PA 19342 (US); LE, Quang, Ngoc, Singapore 9123 (SG); QUINONES, Augusto, Rodolfo, Wilmington, DE 19803 (US)
(74) Representative: Kador & Partner
(86) International application number: US9414168
(87) International publication number: WO9517361

(56) References cited:
- US-A- 3 716 596
- US-A- 5 034 563
- US-A- 5 043 508

## Description

This invention relates to the production of alkylated naphthalenes and substituted naphthalenes.

Alkylaromatic fluids have been proposed for use as certain types of functional fluids where good thermal and oxidative properties are required. For example, U.S. Patent No. 4,714,794 (Yoshida) describes the monoalkylated naphthalenes as having excellent thermal and oxidative stability, low vapor pressure and flash point, good fluidity and high heat transfer capacity and other properties which render them suitable for use as thermal medium oils. The use of a mixture of monoalkylated and polyalkylated naphthalenes as a base for synthetic functional fluids is described in U.S. Patent no. 4,604,491 (Dressler) and Pellegrini U.S. 4,211,665 and 4,238,343 describe the use of alkylaromatics as transformer oils.

The alkylated naphthalenes are usually produced by the alkylation of naphthalene or a substituted naphthalene in the presence of an acidic alkylation catalyst such as a Friedel-Crafts catalyst, for example, an acidic clay as described in Yoshida U.S. 4,714,794 or Dressler U.S. 4,604,491 or a Lewis acid such as aluminum trichloride as described in Pellegrini U.S. 4,211,665 and 4,238,343. The use of a catalyst described as a collapsed silica-alumina zeolite as the catalyst for the alkylation of aromatics such as naphthalene is disclosed in Boucher U.S. 4,570,027. The use of various zeolites including intermediate pore size zeolites such as ZSM-5 and large pore size zeolites such as zeolite L and ZSM-4 for the alkylation of various monocyclic aromatics such as benzene is disclosed in Young U.S. 4,301,316.

In the formulation of functional fluids based on the alkyl naphthalenes, it has been found that the preferred alkyl naphthalenes are the mono-substituted naphthalene since they provide the best combination of properties in the finished product: because the mono-alkylated naphthalenes posses fewer benzylic hydrogens than the corresponding di-substituted or polysubstituted versions, they have better oxidative stability and therefore form better functional fluids and additives. In addition, the mono-substituted naphthalenes have a kinematic viscosity in the desirable range of about 5-8 mm²/s (at 100°C.) when working with alkyl substituents of 14 to 18 carbon atoms chain length. Although the mono-alkylated naphthalenes may be obtained in admixture with more highly alkylated naphthalenes using conventional Friedel-Crafts catalysts such as those mentioned above or by the use of zeolites such as USY, the selectivity to the desired mono-alkylated naphthalenes is not as high as desired.

Several recent advances have been made in this area which improve the yields of the desired mono-alkylated naphthenes.

U.S. Patent 5,034,563, Ashjian et al. teaches use of a zeolite containing a bulky cation. The use of, e.g., USY with cations having a radius of at least 25 nm (2.5 Angstroms) increases selectivity for desired products. Taught as suitable were zeolites containing hydrated cations of metals of Group IA, divalent cations, especially of Group IIA, and cations of the Rare Earths. The patent had examples in which H, NH4, Na were added to USY zeolite by a procedure involving forming a slurry of zeolite and liquid, 1 hour of stirring, decantation, and a repeat of the exchange procedure.

U.S. Patent 5,177,284, Le et al. discusses the desirable properties of alkylated naphthalene fluids with higher alpha:beta ratios, including improved thermal and oxidative stability. Le et al found that several parameters influenced the alpha:beta ratio of the alkylated naphthalene products, including steaming the zeolite, lowering the alkylation temperature; or use of acid-treated clay. Steamed USY catalyst gave excellent results in the examples. The patentees also mentioned use of zeolites with reduced activity due to base exchange, alkaline earth ion exchange and use of boron-zeolite beta.

U.S. Patent 5,191,135 Dwyer et al. discloses the effect of co-feeding water for this reaction when using a large pore zeolite catalyst, such as zeolite Y. Adding from 1 - 3 wt % water to the feed improved the alkylation reaction, a result attributed to suppression of zeolite acid site activity.

U.S. Patent 5,191,134, Le discloses a similar alkylation process using MCM-41.

We did additional work to see if we could further improve this alkylation process. We wanted to increase the efficiency of the reaction both in terms of conversion and yields.

We found that a large pore zeolite, such as zeolite Y, gave unexpected results when incompletely exchanged with rare earths. We achieved very high conversions of both olefin and naphthalene, with essentially all of the product being mono-alkylated, rather than di- or poly-alkylated. Accordingly, the present invention provides a process for preparing long chain alkyl substituted naphthalenes which comprises alkylating a naphthalene with an alkylating agent possessing an alkylating aliphatic group having at least six carbon atoms under alkylation reaction conditions in the presence of an alkylation catalyst comprising a porous crystalline zeolite containing exchangeable site and Rare Earth cations associated with at least some of the exchangeable sites but less than 50 % of the exchangeable sites to form an alkylated naphthalene possessing at least one alkyl group derived from the alkylating agent.

In a more limited embodiment, the present invention provides a process for preparing long chain alkyl substituted naphthalenes which comprises reacting naphthalene with an olefin containing at least 8 carbon atoms as an alkylating agent under alkylation reaction conditions and in the presence of an alkylation catalyst comprising an ultrastable Y zeolite with exchangeable sites containing Rare Earths associated with from 7.5 to 30 % of the exchangeable sites, to form an alkylated naphthalene possessing at least one alkyl group derived from the alkylating agent.

The starting materials for the production of the alkylated naphthalenes are naphthalene and substituted naphthalenes which may contain one or more short chain alkyl groups containing up to eight carbon atoms, such as methyl, ethyl or propyl. Suitable alkyl-substituted naphthalenes include alpha-methylnaphthalene, dimethylnaphthalene and ethylnaphthalene. Naphthalene itself is preferred since the resulting mono-alkylated products have better thermal and oxidative stability than the more highly alkylated materials for the reasons set out above.

The alkylating agents which are used to alkylate the naphthalene include any aliphatic or aromatic organic compound having one or more available alkylating aliphatic groups capable of alkylating the naphthalene . The alkylatable group itself should have at least 6 carbon atoms, preferably at least 8, and still more preferably at least 12 carbon atoms. For the production of functional fluids and additives, the alkyl groups on the alkylnaphthalene preferably have from 12 to 30 carbon atoms, with particular preference to 14 to 18 carbon atoms. A preferred class of alkylating agents are the olefins with the requisite number of carbon atoms, for example, the hexenes, heptenes, octenes, nonenes, decenes, undecenes, dodecenes. Mixtures of the olefins, e.g. mixtures of C₁₂-C₂₀ or C₁₄-C₁₈ olefins, are useful. Branched alkylating agents, especially oligomerized olefins such as the trimers, tetramers, pentamers, etc., of light olefins such as ethylene, propylene, the butylenes, etc., are also useful. Other useful alkylating agents which may be used, although less easily, include alcohols (inclusive of monoalcohols, dialcohols, trialcohols, etc.) such as hexanols, heptanols, octanols, nonanols, decanols, undecanols and dodecanols; and alkyl halides such as hexyl chlorides, octyl chlorides, dodecyl chlorides; and higher homologs.

The alkylation reaction between the naphthalene and the alkylating agent is carried out in the presence of a zeolite catalyst which contains a cation of certain specified radius. The molecular size of the alkylation products will require a relatively large pore size in the zeolite in order for the products to leave the zeolite, indicating the need for a relatively large pore size in the zeolite, which will also tend to reduce diffusion limitations with the long chain alkylating agents. The large pore size zeolites are the most useful zeolite catalysts for this purpose although the less highly constrained intermediate pore size zeolites may also be used, as discussed below. The large pore size zeolites are zeolites such as faujasite, the synthetic faujasites (zeolites X and Y), zeolite L, ZSM-4, ZSM-18, ZSM-20, mordenite and offretite which are generally useful for this purpose and characterized by the presence of a 12-membered oxygen ring system in the molecular structure and by the existence of pores with a minimum dimension of at least 74 nm (7.4 Å), as described by Frilette et al. in J. Catalysis 67,218-222 (1981). See also Chen et al. Shape-Selective Catalysis in Industrial Applications, (Chemical industries; Vol. 36) Marcel Dekker Inc., New York 1989, ISBN 0-8247-7856-1 and Hoelderich et al. Angew. Chem. Int. Ed. Engl. 27 226-246 (1988), especially pp.226-229. The large pore size zeolites may also be characterized by a "Constraint Index" of not more than 2, in most cases not more than 1. Zeolite beta, a zeolite having a structure characterized by twelve-membered pore openings, is included in this class of zeolites although under certain circumstances it has a Constraint Index approaching the upper limit of 2 which is usually characteristic of this class of zeolites. The method for determining Constraint Index is described in U. S. Patent No. 4,016,218, together with values for typical zeolites and of the significance of the Index in U.S. Patent No.4,861,932, to which reference is made for a description of the test procedure and its interpretation.

Zeolites whose structure is that of a ten membered oxygen ring, generally regarded as the intermediate pore size zeolites may also be effective catalysts for this alkylation reaction if their structure is not too highly constrained. Thus, zeolites such as ZSM-12 (Constraint Index 2) may be effective catalysts for this reaction. The zeolite identified as MCM-22 is a useful catalyst for this reaction. MCM-22 is described in U.S. Patent 4,954,325 to which reference is made for a description of this zeolite. Thus, zeolites having a Constraint Index up to 3 will generally be found to be useful catalysts, although the activity may be found to be dependent on the choice of alkylating agent, especially its chain length, a factor which imposes diffusion limitations upon the choice of zeolite.

A highly useful zeolite for the production of the mono-alkylated naphthalenes is zeolite Y in the ultrastable form, usually referred to as USY. When this material contains hydrated cations, it catalyses the alkylation in good yields with excellent selectivity. Zeolite USY is a material of commerce, available in large quantities as a catalyst for the cracking of petroleum. It is produced by the stabilization of zeolite Y by a procedure of repeated ammonium exchange and controlled steaming. Processes for the production of zeolite USY are described in U. S. Patents Nos. 3,402,966 (McDaniel), 3,923,192 (Maher) and 3,449,070 (McDaniel); see also Wojciechowski, Catalytic Cracking, Catalysts, Chemistry and Kinetics, (Chemical Industries Vol. 25), Marcel Dekker, New York, 1986, ISBN 0-8247-7503-8, to which reference is made for a description of zeolite USY, its preparation and properties.

We prefer to use a small crystal Y zeolite, in the 0.2 to 0.4 µm range, although the 0.6 to 1.3 µm material which is more typical of Y zeolite crystals may also be used.

### Rare Earth Exchange

The selected zeolite catalyst contains a critical amount of one or more of the Rare Earths. Suitable are Y, La and any of the Lanthanum Series of Rare Earths, Ce, Pr, Nd, Pm, Sm, Eu, Gd, Tb, Dy, Ho, Er, Tm Yb, and Lu. Especially preferred are Ce, Y and La. In most applications, mixtures of rare earths will be preferred, as these are readily available commercially and much less expensive than purified rare earth elements. The mixed rare earths typically used to produce zeolite Y based cracking catalysts serve well.

If the zeolite does not contain the desired amount of rare earths they may be introduced by ion-exchange in the conventional manner using a solution of the exchanging cation.

The amount of rare earth exchange is critical. Although essentially complete exchange, such as that obtained in the two step exchange procedure of U.S. Patent 5,034,563, gives a catalyst that will work, complete exchange does not give optimum results.

We discovered that it is important to have less than 50 % of the total number of exchangeable sites associated with Rare Earths (RE). Preferably less than 35 % of the exchangeable sites contain Rare Earths, and most preferably less than 20 % exchange is preferred. Optimum results are seen with 12.5 to 17.5 % Rare Earth exchange, ideally, with 15 % exchange. As used herein, the total ion exchange capacity of the catalyst may be determined by the temperature programmed ammonia desorption method described by G. T. Kerr and A. W. Chester in Thermochimica Acta, 3, 113-124 (1971).

For a typical catalyst, comprising 20 - 50 wt % USY zeolite in a conventional binder or matrix as discussed below, the optimum RE content will be 1 wt %, equivalent to a rare earth content corresponding to less than 15 % of the total number of exchangeable sites. RE contents may range from 0.025 to 2.5 wt %, preferably 0.05 to 2 wt %, and most preferably, 0.2 to 1.5 wt %.

Expressed in terms of the role the Rare Earths play in our catalyst, we believe the RE content is in the sodalite cages, not in the supercage of the zeolite. The '563 patent was directed to bulky cations in the supercage. When our low RE catalyst is calcined, the RE is believed to go into the sodalite cage. Commercially, such partial exchange is easy to do, a solution with the desired amount of RE may be simply sprayed onto a moving bed or layer of suitable zeolite, either neat zeolite or composited with a matrix.

Binders may be incorporated to improve crush strength and other physical properties. Suitable materials include naturally occurring clays, e.g., bentonite and kaolin as well as the oxides referred to above, silica, alumina, and mixtures thereof.

The relative proportions of zeolite, present in finely divided crystalline form oxide matrix may vary widely, with the crystalline zeolite content ranging from 1 to 90 percent by weight and more usually, particularly when the composite is prepared in the form of beads, in the range of 2 to 80 weight percent of the composite.

The stability of the alkylation catalyst of the invention may be increased by steaming. U.S. Patent Nos. 4,663,492; 4,594,146; 4,522,929; and 4,429,176, describe conditions for the steam stabilization of zeolite catalysts which can be utilized to steam-stabilize the catalyst. The steam stabilization conditions include contacting the catalyst with, e.g., 5-100% steam at a temperature of at least 300°C (e.g., 300-650°C) for at least one hour (e.g., 1-200 hours) at a pressure of 100 - 2,500 kPa, e.g. steaming with 75-100% steam at 315°-500°C and atmospheric pressure for 2-25 hours. The steam stabilization treatment may, as described in the above-mentioned patents, take place under conditions sufficient to initially increase the Alpha Value of the catalyst, and produce a steamed catalyst having a peak Alpha Value. If desired, steaming can be continued to subsequently reduce the Alpha Value from the peak Alpha Value to an Alpha Value which is substantially the same as the Alpha Value of the unsteamed catalyst.

The catalyst performance may be further improved by partial ammonium exchange, e.g., ammonium exchanged and then calcined at 400°C (752°F) to remove only some of the ammonia.

### Alkylation Process Conditions

Conventional alkylation conditions and equipment can be used. These will be briefly reviewed for completeness, but these conditions, per se, form no part of the claimed invention.

The alkylation process of this invention is conducted such that the organic reactants, i.e., the alkylatable aromatic compound and the alkylating agent, are brought into contact with the zeolite catalyst in a suitable reaction zone such as, for example, in a flow reactor containing a fixed bed of the catalyst composition, under effective alkylation conditions. Such conditions typically include a temperature of from 100°C to 400°C, a pressure of from 20.3 to 25325 kPa (0.2 to 250 atmospheres), a feed weight hourly space velocity (WHSV) of from about 0.1 hr⁻¹ to 10 hr⁻¹ and an alkylatable aromatic compound to alkylating agent mole ratio of from 0.1:1 to 50:1, preferably from 4:1 to 1:4 e.g. from 2:1 to 1:2. The WHSV is based upon the weight of the catalyst composition employed, i.e., the total weight of active catalyst (and binder if present).

Preferred reaction conditions include a temperature within the approximate range of from 100°C to 350°C, a pressure of from 101 to 2533 kPa (1 to 25 atmospheres), a WHSV of from 0.5 hr⁻¹ to 5 hr⁻¹ and an alkylatable aromatic compound to alkylating agent mole ratio of from 0.5:1 to 5:1. When using naphthalene as the aromatic compound, the pressure should preferably be maintained at a value of at least 350 kPa gauge (50 psig) in order to prevent the naphthalene from subliming into the overhead of the alkylation reactor; the required pressure may be maintained by inert gas pressurization, preferably with nitrogen. The reactants can be in either the vapor phase or the liquid phase and can be neat, i.e., free from intentional admixture or dilution with other material, or they can be brought into contact with the zeolite catalyst composition with the aid of carrier gases or diluents such as, for example, hydrogen or nitrogen. The alkylation can be carried out as a batch-type reaction typically employing a closed, pressurized, stirred reactor with an inert gas blanketing system or in a semi-continuous or continuous operation utilizing a fixed or moving bed catalyst system.

The presence of some water in the feed may improve selectivity, e.g., operation with water addition to the feed or hydration of the catalyst. In a fluid bed reactor operating with 0.75 wt % water in the reaction mixture is preferred.

The products comprising alkylated aromatics are characterized by exceptional oxidative and thermal stability. They may be separated from the reaction mixture by stripping off unreacted alkylating agent and naphthalene compound in the conventional manner. It has also been found that the stability of the alkylated product may be improved by filtration over activated charcoal and by alkali treatment to remove impurities, especially acidic by-products formed by oxidation during the course of the reaction. The alkali treatment is preferably carried out by filtration over a solid alkali material, preferably calcium carbonate (lime). In a typical product work-up, it has been found, for example, that the RBOT (Rotating Bomb Oxidation Test) stability can be increased from a value of 184 minutes for an unstripped product (C₁₄-alkylnaphthalene) to 290 minutes if the unreacted materials are removed by stripping and to 350 minutes if the stripped product is filtered over lime (CaCO₃).

### EXAMPLE 1

A commercially available catalyst containing 40 wt.% USY (Unit Cell Size = 244.5 nm (24.45 A)) in a clay and silica sol matrix was ammonium exchanged at room temperature by slurrying the catalyst with 5 vol/vol of 1 N NH₄NO₃ for one hour. The catalyst was washed with water and then reexchanged and washed using the same procedure. This catalyst was then.calcined for 5 hours in flowing air at a series of temperatures ranging from 350°C to 538°C to produce catalysts containing varying amounts of ammonia.

The relative amounts of cation exchange sites in the ammonium and protonic form are shown below:

| Catalyst ID | Calcination Temperature, °C | NH₄⁺ Conc. (meq/g) | H⁺ Conc. (meq/g) |
|---|---|---|---|
| A | None | 1.09 | -0- |
| B | 350 | 0.85 | 0.24 |
| C | 375 | 0.64 | 0.45 |
| D | 400 | 0.59 | 0.50 |
| E | 425 | 0.54 | 0.55 |
| F | 450 | 0.34 | 0.75 |
| G | 538 | -0- | 1.09 |

The total ion exchange capacity of the catalyst as determined by temperature programmed ammonia desorption (Chester and Kerr method) is 1.09 meq/g.

### EXAMPLE 2

In a series of six experiments, five parts of each of the catalysts of Example 1 were combined with ninety-five parts of naphthalene and 1-hexadecene in a 1:1.2 molar ratio in a stirred vessel. The contents of the vessel were then heated to 200°C and held at this temperature for four hours. The contents of the vessel were analyzed using gas chromatography to determine the amounts of unreacted naphthalene, olefin, monoalkylate and dialkylate. The results are summarized below:

| Catalyst ID | Naphthalene (wt.%) | Hexadecene (wt.%) | Monoalkylate (wt.%) | Dialkylate (wt.%) |
|---|---|---|---|---|
| A | 32.3 | 67.7 | 0 | 0 |
| B | 20.3 | 42.6 | 37.0 | 0 |
| C | 15.5 | 35.7 | 48.3 | 0.5 |
| D | 6.3 | 19.2 | 72.9 | 1.6 |
| E | 13.5 | 30.1 | 55.4 | 1.0 |
| F | 12.2 | 29.6 | 56.6 | 1.6 |
| G | 12.9 | 27.7 | 59.1 | 0.3 |

### EXAMPLE 3

A commercially available USY with the properties shown below was combined with kaolin clay and a colloidal silica (Nalco) and spray dried at a temperature of 177°C (350°F) to produce a fluid catalyst containing 40 wt.% USY.

| | |
|---|---|
| UC Lattice Parameter, A | 24.29 |
| Surface Area, m²/g | 650 |
| Sodium, ppm | 445 |
| SiO₂, wt.% | 85.8 |
| Al₂O₃, wt.% | 10.8 |
| SiO₂/Al₂O₃ (molar) | 13.8 |
| Ash at 1000°C | 98.7 |
| Real Density, g/ml | 2.384 |

| Sorption Capacities** | |
|---|---|
| n-C₆ (p = 40 torr) | 21.1 |
| Cy-C₆ (p = 40 torr) | 19.8 |
| H₂O (p = 12 torr) | 10.6 |

| | |
|---|---|
| * Surface area measured at P/Pₒ = 0.03 ** Sorption Capacities are shown as wt ratios x 100 (= wt of sorbate x 100/wt of sorbate free zeolite) | |

This catalyst was ion exchanged with ammonium nitrate using the procedure of Example 1 then calcined at 400°C in flowing air for five hours to produce Catalyst H. Another catalyst, Catalyst I, was prepared from the same base ammonium exchanged material by calcining at 538°C in flowing air for five hours.

The ammonia content and water content of these catalysts were analyzed by TPAD and by determination of loss on ignition at 700°C. The results are summarized below:

| Catalyst ID | Calcination Temperature, °C | NH₄⁺ Conc. (meq/g) | H⁺ Conc. (meq/g) | H₂O Conc. (wt.%) |
|---|---|---|---|---|
| H | 400 | 0.04 | 0.06 | 2.0 |
| I | 538 | -0- | 0.10 | 0.1 |

### EXAMPLE 4

Five parts of each of the catalysts of Example 3 were combined with ninety-five parts of naphthalene and 1-hexadecene in a 1:1.2 molar ratio in a stirred vessel. The contents of the vessel were then heated to 200°C and held at this temperature for four hours. The contents of the vessel were analyzed using gas chromatography to determine the amounts of unreacted naphthalene, olefin, monoalkylate and dialkylate.

The results are summarized below:

| Catalyst ID | Naphthalene (wt.%) | Hexadecene (wt.%) | Monoalkylate (wt.%) | Dialkylate (wt.%) |
|---|---|---|---|---|
| H | 3.1 | 8.3 | 79.7 | 8.9 |
| I | 4.9 | 5.9 | 63.0 | 26.2 |

### EXAMPLE 5

Five parts of Catalyst J, a commercially available rare earth containing USY (REUSY) catalyst having the properties shown below were combined with ninety-five parts of naphthalene and 1-hexadecene in a 1:1.2 molar ratio in a stirred vessel.

| Properties of the Catalyst Used in This Example | |
|---|---|
| RE₂O₃ content, wt.% | 1.0 |
| Unit Cell Lattice Parameter, nm(A) | 245.7 (24.57) |
| Ammonia Content, meq/g | 0.56 |
| Ash, wt.% (at 700°C) | 89.5 |

The contents of the vessel were then heated to 200°C and held at this temperature for four hours. The contents of the vessel were analyzed using gas chromatography to determine the amounts of unreacted naphthalene, olefin, monoalkylate and dialkylate. The results are summarized below:

| Catalyst ID | Naphthalene (wt.%) | Hexadecene (wt.%) | Monoalkylate (wt.%) | Dialkylate (wt.%) |
|---|---|---|---|---|
| J | 2.5 | 13.6 | 82.1 | 1.8 |

### EXAMPLE 6

Five parts of Catalyst K, a commercially available rare earth containing USY (REUSY) catalyst having the properties shown below were combined with ninety-five parts of naphthalene and 1-hexadecene in a 1:1.2 molar ratio in a stirred vessel.

| Properties of the Catalyst Used in This Example | |
|---|---|
| RE₂O₃ content, wt.% | 3.0 |
| Unit Cell Lattice Parameter, nm(A) | 245.7(24.57) |
| Ammonia Content, meq/g | 0.50 |
| Ash, wt.% (at 700°C) | 80 |

The contents of the vessel were then heated to 200°C and held at this temperature for four hours. The contents of the vessel were analyzed using gas chromatography to determine the amounts of unreacted naphthalene, olefin, monoalkylate and dialkylate. The results are summarized below:

| Catalyst ID | Naphthalene (wt.%) | Hexadecene (wt.%) | Monoalkylate (wt.%) | Dialkylate (wt.%) |
|---|---|---|---|---|
| K | 24.3 | 50.2 | 25.0 | 0.5 |

Comparison of the results of Examples 5 and 6 show that the rare earth content of the catalysts can affect yields and that the lower rare earth concentration (i.e., 1 wt.%) is preferred. The rare earth content of the preferred catalyst (Example 5) would correspond to less than 15% of the total number of exchangeable sites. This demonstrates the importance of partial Rare Earth exchange.

While we do not know exactly why our catalyst works so much better than the prior art catalyst with more RE, we believe the difference may be due to the fate of RE. We believe that the RE in our new catalyst, e.g., the USY with 1 wt % RE, is in the sodalite cages, not in the supercage. The '563 patent was directed to bulky cations in the supercage. When our low RE catalyst is calcined, the RE goes into the sodalite cage. Preferably most of the rare earths are in the supercage, and ideally essentially all of the rare earths are in the supercage.

Commercially partial exchange is easy to achieve, a solution with the desired amount of RE may be simply sprayed on the zeolite or the catalyst in the desired amount.

Results may be further improved by ammonium exchange followed by calcination to convert no more than about half of the ammonium form to the protonic form. It is also beneficial to have some water present, but the amount of water found in most commercial grade naphthalene supplies (typically 0.75 wt %) is enough.

## Claims

1. A process for preparing long chain alkyl substituted naphthalenes which comprises alkylating a naphthalene with an alkylating agent possessing an alkylating aliphatic group having at least six carbon atoms under alkylation reaction conditions in the presence of an alkylation catalyst comprising a porous crystalline zeolite containing exchangeable site and Rare Earth cations associated with at least some of the exchangeable sites but less than 50 % of the exchangeable sites to form an alkylated naphthalene possessing at least one alkyl group derived from the alkylating agent.

2. The process of Claim 1 wherein the zeolite is a large pore size zeolite having pores with a minimum dimension of at least 74 nm.

3. The process of Claim 1 wherein the zeolite has a Constraint Index of not more than 2.

4. A process according to Claim 3 wherein the zeolite has a Constraint Index of not more than 1.

5. The process of Claim 1 wherein the zeolite comprises zeolite X or zeolite Y.

6. The process of Claim 5 wherein the zeolite is USY.

7. The process of Claim 1 wherein Rare Earths are associated with 5 to 35 % of the exchangeable sites.

8. The process of Claim 1 wherein Rare Earths are associated with 7.5 to 30 % of the exchangeable sites.

9. The process of Claim 1 wherein Rare Earths are associated with 10 to 20 % of the exchangeable sites.

10. The process of Claim 1 wherein the alkylating aliphatic group contains at least 8 carbon atoms.

11. The process of Claim 1 wherein the alkylating aliphatic group contains at least 12 carbon atoms.

12. The process of Claim 1 wherein the alkylating aliphatic group contains from 14 to 20 carbon atoms.

13. The process of Claim 1 wherein the alkylating agent comprises an olefin.

14. The process of Claim 1 wherein the alkylation reaction conditions include a temperature of 100°C to 400°C, a pressure of 20.3 to 25325 kPa, a weight hourly space velocity (WHSV) of 0.1 to 10 and an alkylatable aromatic compound to alkylating agent mole ratio of from 0.1:1 to 50:1.

15. The process of Claim 14 wherein the alkylation reaction conditions include a temperature of 100°C to 300°C, a pressure of 101 to 2533 kPa, a WHSV of 0.5 to 5 and an alkylatable aromatic compound to alkylating agent mole ratio of 0.5:1 to 5:1.

16. The process of claim 6 wherein the catalyst contains: 20 to 50 % ultrastable Y zeolite, and 0.2 to 2.0 wt % rare earths.

17. The process of claim 16 wherein the catalyst contains 1.0 wt % rare earths.

## Patentansprüche

1. Verfahren zur Herstellung von mit langkettigen Alkylgruppen substituierten Naphthalinen, das das Alkylieren von Naphthalin mit einem Alkylierungsmittel, das eine alkylierende aliphatische Gruppe mit mindestens sechs Kohlenstoffatomen besitzt, bei Alkylierungsreaktionsbedingungen in Gegenwart eines Alkylierungskatalysators umfaßt, der einen porösen kristallinen Zeolith umfaßt, der einen Austauschplatz enthält und bei dem Kationen der Seltenen Erden mit zumindest einigen Austauschplätzen, jedoch mit weniger als 50% der Austauschplätze verbunden sind, wodurch alkyliertes Naphthalin hergestellt wird, das mindestens eine Alkylgruppe besitzt, die vom Alkylierungsmittel stammt.

2. Verfahren nach Anspruch 1, wobei der Zeolith ein großporiger Zeolith mit Poren mit einer Mindestabmessung von mindestens 74 nm ist.

3. Verfahren nach Anspruch 1, wobei der Zeolith einen Zwangsindex von nicht mehr als 2 hat.

4. Verfahren nach Anspruch 3, wobei der Zeolith einen Zwangsindex von nicht mehr als 1 hat.

5. Verfahren nach Anspruch 1, wobei der Zeolith Zeolith X oder Zeolith Y umfaßt.

6. Verfahren nach Anspruch 5, wobei der Zeolith USY ist.

7. Verfahren nach Anspruch 1, wobei die Seltenen Erden mit 5 bis 35% der Austauschplätze verbunden sind.

8. Verfahren nach Anspruch 1, wobei die Seltenen Erden mit 7,5 bis 30% der Austauschplätze verbunden sind.

9. Verfahren nach Anspruch 1, wobei die Seltenen Erden mit 10 bis 20% der Austauschplätze verbunden sind.

10. Verfahren nach Anspruch 1, wobei die alkylierende aliphatische Gruppe mindestens 8 Kohlenstoffatome enthält.

11. Verfahren nach Anspruch 1, wobei die alkylierende aliphatische Gruppe mindestens 12 Kohlenstoffatome enthält.

12. Verfahren nach Anspruch 1, wobei die alkylierende aliphatische Gruppe 14 bis 20 Kohlenstoffatome enthält.

13. Verfahren nach Anspruch 1, wobei das Alkylierungsmittel ein Olefin umfaßt.

14. Verfahren nach Anspruch 1, wobei die Alkylierungsreaktionsbedingungen eine Temperatur von 100 bis 400°C, einen Druck von 20,3 bis 25.325 kPa, eine stündliche Gewichts-Raum-Geschwindigkeit (WHSV) von 0,1 bis 10 und ein Molverhältnis von alkylierbarer aromatischer Verbindung zu Alkylierungsmitteln von 0,1:1 bis 50:1 umfassen.

15. Verfahren nach Anspruch 14, wobei die Alkylierungsreaktionsbedingungen eine Temperatur von 100 bis 300°C, einen Druck von 101 bis 2.533 kPa, eine WHSV von 0,5 bis 5 und ein Molverhältnis von alkylierbarer aromatischer Verbindung zu Alkylierungsmittel von 0,5:1 bis 5:1 umfassen.

16. Verfahren nach Anspruch 16, wobei der Katalysator enthält: 20 bis 50% ultrastabilen Zeolith Y und 0,2 bis 2,0 Gew.-% Seltene Erden.

17. Verfahren nach Anspruch 16, wobei der Katalysator 1,0 Gew.-% Seltene Erden enthält.

## Revendications

1. Un procédé de préparation de naphtalène substitué par un alkyle à chaîne longue, qui comprend l'alkylation du naphtalène par un agent d'alkylation possédant un groupe aliphatique alkylant ayant au moins six atomes de carbone, dans des conditions de réaction d'alkylation en présence d'un catalyseur d'alkylation comprenant une zéolite cristalline poreuse contenant des sites échangeables et des cations des terres rares associés avec au moins une partie des sites échangeables, mais moins de 50% des sites échangeables pour former un naphtalène alkylé possédant au moins un groupe alkyle dérivant de l'agent d'alkylation.

2. Le procédé selon la revendication 1, dans lequel la zéolite est une zéolite à grande taille de pores, ayant des pores d'une dimension minimum d'au moins 74 nm.

3. Le procédé selon la revendication 1, dans lequel la zéolite présente un indice de contrainte non supérieur à 2.

4. Le procédé selon la revendication 3, dans lequel la zéolite présente un indice de contrainte non supérieur à 1.

5. Le procédé selon la revendication 1, dans lequel la zéolite comprend la zéolite X ou la zéolite Y.

6. Le procédé selon la revendication 5, dans lequel la zéolite est une USY.

7. Le procédé selon la revendication 1, dans lequel les terres rares sont associées avec 5 à 35% des sites échangeables.

8. Le procédé selon la revendication 1, dans lequel les terres rares sont associées avec 7,5 à 30% des sites échangeables.

9. Le procédé selon la revendication 1, dans lequel les terres rares sont associées avec 10 à 20% des sites échangeables.

10. Le procédé selon la revendication 1, dans lequel le groupe aliphatique alkylant contient au moins 8 atomes de carbone.

11. Le procédé selon la revendication 1, dans lequel le groupe aliphatique alkylant contient au moins 12 atomes de carbone.

12. Le procédé selon la revendication 1, dans lequel le groupe aliphatique alkylant contient de 14 à 20 atomes de carbone.

13. Le procédé selon la revendication 1, dans lequel l'agent alkylant comprend une oléfine.

14. Le procédé selon la revendication 1, dans lequel les conditions de la réaction d'alkylation comprennent une température de 100°C à 400°C, une pression de 20,3 à 25325 kPa, une vitesse spatiale horaire pondérale (WHSV) de 0,1 à 10 et un rapport molaire composé aromatique alkylable/agent alkylant de 0,1/1 à 50/1.

15. Le procédé selon la revendication 14, dans lequel les conditions de la réaction d'alkylation comprennent une température de 100°C à 300°C, une pression de 101 à 2533 kPa, une WHSV de 0,5 à 5 et un rapport molaire composé aromatique alkylable/agent alkylant de 0,5/1 à 5/1.

16. Le procédé selon la revendication 6, dans lequel le catalyseur contient : 20 à 50% de zéolite y ultra stable et
0,2 à 2,0% en poids de terres rares.

17. Le procédé selon la revendication 16, dans lequel le catalyseur contient 1,0% de terres rares.
